(19) Europäisches Patentamt
European Patent Office
Office européen des brevets

(11) Veröffentlichungsnummer : **0 226 043**
**B1**

(12) # EUROPÄISCHE PATENTSCHRIFT

(45) Veröffentlichungstag der Patentschrift : **14.02.90**

(21) Anmeldenummer : **86115683.4**

(22) Anmeldetag : **12.11.86**

(51) Int. Cl.[5] : **C 07 B 55/00**, C 07 B 57/00, C 07 C 67/10// C07D233/32, C07C69/74, C07C69/34

(54) **Verfahren zur Herstellung von Enantiomeren.**

(30) Priorität : **23.11.85 DE 3541450**

(43) Veröffentlichungstag der Anmeldung : **24.06.87 Patentblatt 87/26**

(45) Bekanntmachung des Hinweises auf die Patenterteilung : **14.02.90 Patentblatt 90/07**

(84) Benannte Vertragsstaaten : **AT BE CH DE FR GB IT LI NL SE**

(56) Entgegenhaltungen :
**EP–A– 0 050 074**
**EP–A– 0 173 185**
**METHODEN DER ORGANISCHEN CHEMIE, HOUBEN-WEYL, Band E5, 1985, Seiten 702-703, Georg Thieme Verlag Stuttgart, New York, US**
**Die Akte enthält technische Angaben, die nach dem Eingang der Anmeldung eingereicht wurden und die nicht in dieser Patentschrift enthalten sind.**

(73) Patentinhaber : **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder : **Casutt, Michael, Dr.**
**Adolf-Kolping-Strasse 19**
**D-6102 Pfungstadt (DE)**
Erfinder : **Poetsch, Eike, Dr.**
**Am Buchwald 4**
**D-6109 Mühltal 6 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 226 043 B1

**Beschreibung**

Die Erfindung betrifft ein Verfahren zur Herstellung von Enantiomeren von optisch aktiven Dicarbonsäuremonoestern durch intramolekulare Umlagerung.

Enantiomere von optisch aktiven Dicarbonsäuremonoestern sind wertvolle Zwischenprodukte bei der Herstellung optisch aktiver Verbindungen wie beispielsweise von (—)-Pantolacton (Seebach, Wasmuth ; Helv. Chim. Acta 63 (1980), 197) oder des Pheromons Anthopleurin (Musich, Rapoport ; J. Amer. Chem. Soc. 100 (1978), 4865).

Es ist eine Reihe von Verfahren zur Herstellung von enantiomeren Dicarbonsäuremonoestern bekannt. So lassen sich Dicarbonsäurediester partiell chemisch hydrolysieren (z. B. Durham et al. in Org. Synth. Coll. Vol. IV, Wiley, New York, 1963, S. 635).

Enzymatische Hydrolyseverfahren, wie beispielsweise in EP-A-0 084 892 und JP-A-57 198 098 offenbart, liefern direkt ein Enantiomeres. Aus Dicarbonsäureanhydriden werden durch Alkoholyse ebenfalls Monoester erhalten (Houben-Weyl, Methoden der Organischen Chemie, Georg-Thieme-Verlag, Stuttgart, Bde. VIII + E5). Die nach den voranstehenden Verfahren gewonnenen Dicarbonsäuremonoester können nach bekannten Methoden zur Racematspaltung (z. B. in P. Newman : Optical Resolution Procedures for Chemical Compounds, Vol. 2, Optical Resolution Information Center, Manhattan College, Riverdale, New York, 1981), beispielsweise über die Bildung diastereomerer Salze wie in EP-A-0 092 194 oder DE-A-34 294 beschrieben, in die optischen Antipoden gespalten werden.

Diese bisher bekannten Verfahren weisen eine Reihe von Nachteilen auf. So kann aus dem racemischen Gemisch der enantiomeren Dicarbonsäuremonoester bestenfalls nur die Hälfte der in das Trennverfahren eingesetzten Gesamtmenge an gewünschtem Enantiomeren gewonnen werden. Das werbleibende unerwünschte Enantiomere muß wieder in eine racemische, der optischen Trennung zugängliche Form überführt werden. Dies kann beispielsweise durch Veresterung zu dem entsprechenden Dicarbonsäurediester geschehen, der erneut partiell hydrolysiert werden kann. Weiterhin kann der unerwünschte optisch aktive Halbester vollständig zur Dicarbonsäure verseift werden und diese nach Überführung in das Anhydrid mittels eines Alkohols erneut in ein Gemisch optisch trennbarer Halbester umgesetzt werden.

Zur vollständigen Umwandlung des unerwünschten in das gewünschte Enantiomere müssen die Prozesse der optischen Trennung und Rückführung in eine Racematform gegebenenfalls mehrfach ausgeführt werden.

Es besteht somit ein Bedürfnis nach einem Verfahren, das die Umwandlung optisch aktiver Halbester von Dicarbonsäuren ineinander ohne zusätzliche Verfahrensschritte bewirkt und damit die einfache Herstellung von Enantiomeren dieser Halbester erlaubt.

Diese Aufgabe wird gelöst durch das erfindungsgemäße Verfahren zur Umwandlung eines Enantiomeren eines optisch dessen zugrundeliegende Dicarbonsäure eine Spiegelebene im Molekül aufweist, aktiven Dicarbosäuremonoesters dessen zugrundeliegende Dicarbonsäure eine Spiegelebene im Molekül aufweist, in das Enantiomere mit umgekehrten Drehsinn durch Racemisierung und nachfolgende Trennung, dadurch gekennzeichnet, daß man das eingesetzte Enantiomere einer Umesterung unter Protonensäure — oder Nebengruppenmetallsalz-Katalyse unterwirft und das gewünschte Enantiomere aus dem Racemat entweder durch direkte Kristallisation oder nach Überführung mittels einer optisch aktiven Base in ein Diastereomerensalzpaar und dessen Trennung gewinnt.

Die intermolekulare Umlagerung von Carbonsäuren und Estern unterschiedlicher Carbonsäuren ist bekannt (vgl. Houben-Weyl, a.a.O.). Bei dieser Reaktion erhaltene Gleichgewichtsgemische lassen sich oftmals durch Entfernen eines Reaktionspartners in Richtung eines Produktes beeinflussen.

Die intermolekulare Umlagerung unter Nebengruppenmetallsalz-Katalyse wird z. B. von P.D. Jeffrey, S.W. Mc Combie in J. Am. Chem. Soc. 1982, 47, 587-590 und D. Häbich, P. Naab, K. Metzger in Tetrahedron Letters Vol. 24, No. 25, 2559-2562 (1983) beschrieben.

Monoester aus achiralen Dicarbonsäuren und optisch aktiven Alkoholen erleiden unter Acidolysebedingungen eine Racemisierung im Alkoholteil (Balfe et al., J. Chem. Soc. 1946, 803).

In der EP-A2-00 50 074 wird die Isomerisierung des 1-Monomethylesters der (1R, 3S) cis 2,2-Dimethylcyclopropan-1-3-dicarbonsäure zur trans (1S, 3S)-Verbindung mit Natriumalkoholat beschrieben. Hierbei tritt jedoch keine Umesterung, sondern eine Inversion am Chiralitätszentrum ein.

Aufgrund des bei einer intramolekularen Umlagerung des im Carbonsäureteil optisch aktiven Dicarbonsäuremonoesters zu erwartenden Produktgemisches, war es daher umso überraschender, daß die Umlagerung der Monoester praktisch einheitlich ohne merkliche Bildung von Nebenprodukten erfolgt.

Für die Anwendung in dem erfindungsgemäßen Verfahren besonders geeignet sind die für eine Umesterung bekannten Bedingungen. Die Übertragung des Alkoholrestes wird durch $H^+$-Ionen oder Salze von Nebengruppenmetallen katalysiert.

Als Katalysatoren eignen sich Protonensäuren wie Schwefelsäure, Phosphorsäure, Perchlorsäure, Methansulfonsäure, Toluolsulfonsäure oder Borsäure. Nebengruppenmetallsalze wie Triphenylphosphinpalladium (O)- und -nickel (O)-komplexe, ferner Alkoxytrialkylzinnverbindungen oder Titan (IV)-alkoholate.

Die Ausführung der Reaktion gestaltet sich einfach. Als umzulagernde Enantiomere setzt man die optisch aktiven Monoester oder deren Enantiomerengemische von offenkettigen oder cyclischen 1,ω-Dicarbonsäuren ein.

Im Falle des Vorliegens von Spiegelebenen in den Formeln der den Monoestern zugrundeliegenden Dicarbonsäuren liegen Enantiomerenpaare mit betragsmäßig gleichem Drehsinn für die jeweiligen Antipoden vor, die den allgemeinen Formeln I bis III entsprechen, wobei

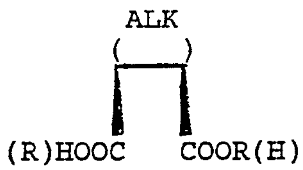
I

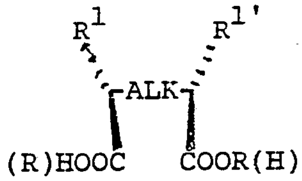
II

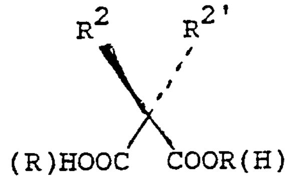
III

R den Rest eines zur Bildung der Monoester geeigneten Alkohols,

ALK eine gegebenenfalls in Bezug auf die Carbonylgruppen symmetrisch substituierte Kohlenstoffkette und

$R^1$, $R^{1'}$, $R^2$, $R^{2'}$ organische, von Wasserstoff verschiedene Reste bedeuten, mit der Maßgabe, daß $R^1$ und $R^{1'}$ gleich, $R^2$ und $R^{2'}$ jedoch verschieden sind.

Unter Substitution im Strukturelement ALK wird auch der Ersatz einer oder mehrerer $CH_2$-Gruppen durch Heteroatome wie O, N oder S, durch —CH = CH— oder —C ≡ C-Gruppen oder durch carbocyclische oder heterocyclische Ringe verstanden.

Beispielsweise treten die von den Grundstrukturen a bis g abgeleiteten Dicarbonsäuremonoester als Enantiomere auf und können nach dem erfindungsgemäßen Verfahren ineinander umgewandelt werden :

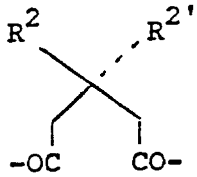
a

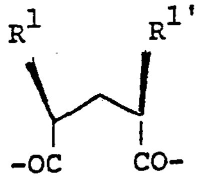
b

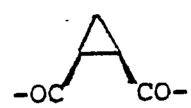
c

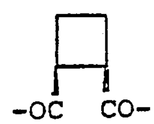
d

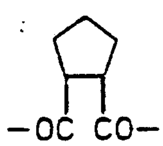
e

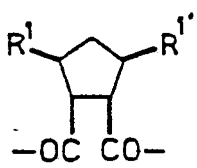
f

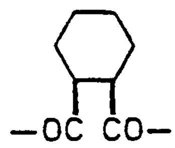
g

Vorzugsweise eignen sich Dicarbonsäuremonoester mit cyclischer Struktur zur Umwandlung nach dem erfindungsgemäßen Verfahren, insbesondere solche mit drei-, vier-, fünf- und sechsgliedrigen Ringen.

Die Art der die Halbester bildenden Alkohole ist im Rahmen der vorliegenden Erfindung nicht kritisch. Es können sowohl offenkettige als auch cyclische Alkohole eingesetzt werden. Vorzugsweise trifft man die Wahl aus der Gruppe der primären Alkohole.

Die Umlagerung der optisch aktiven Dicarbonsäuremonoester erfolgt zweckmäßig in einem inerten organischen Lösungsmittel, wobei sich der Racemisierungsgrad als lösungsmittelunabhängig erweist; einzig der für die Gleichgewichtseinstellung erforderliche Zeitraum wird durch die Art des Solvents beeinflußt. Gut geeignet sind insbesondere Ether wie Diethylether, Di-n-butylether, Tetrahydrofuran, Dioxan oder Anisol, Ketone wie Aceton, Butanon oder Cyclohexanon, Amide wie Dimethylformamid oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Die Umlagerung kann auch in Abwesenheit eines Lösungsmittels, z. B. durch einfaches Erhitzen der Komponenten in Gegenwart eines geeigneten Katalysators, durchgeführt werden. Die Reaktionstemperatur liegt gewöhnlich zwischen — 50° und + 250°, vorzugsweise zwischen 0° und 120°. Bei diesen Temperaturen sind die Umlagerungen in der Regel nach 15 Minuten bis 48 Stunden beendet.

Die Ausführung der Umlagerung erfolgt bevorzugt durch Lösen des zu racemisierenden Dicarbonsäuremonoesters bzw. des Gemisches seiner Enantiomeren in einem geeigneten Lösungsmittel, Hinzufügen des Katalysators und Erwärmen des Reaktionsgemisches auf die zur Umlagerung erforderliche Temperatur, wobei während der Reaktionszeit zweckmäßigerweise gerührt wird.

Zur Aufarbeitung nach Durchführung der Reaktion entfernt man zweckmäßigerweise das Lösungsmittel und unterwirft den Rückstand einem für optisch aktive Carbonsäuren geeigneten Spaltungsverfahren (wie z. B. angegeben bei P. Newman, ibid.).

Vorzugsweise überführt man das erhaltene Racemat mittels einer optisch aktiven Base in ein Gemisch diastereomerer Salze, die man beispielsweise durch Chromatographie, extraktive Verteilung, mechanische Auslese, insbesondere jedoch durch Kristallisation trennt. Aus den isolierten Salzen erhält man den gewünschten optisch aktiven Dicarbonsäuremonoester, beispielsweise durch Freisetzen mit Säuren, insbesondere mittels verdünnter Mineralsäuren.

In einer bevorzugten Ausführungsform der vorliegenden Erfindung führt man die Kristallisation des gewünschten Enantiomeren oder eines seiner Salze während der Umesterung durch und überführt damit den eingesetzten Monoester vollständig in das gewünschte Enantiomere.

So kristallisiert beispielsweise aus bei der zur Durchführung der Umlagerung erforderlichen erhöhten Temperatur gesättigten Lösungen diastereomerer Salze optisch aktiver Dicarbonsäuremonoester beim Abkühlen ein optisch einheitliches Salz. Nach dessen Abtrennung wird die Reaktionslösung bei erhöhter Temperatur erneut mit dem umzulagernden diastereomeren Salz des Enantiomeren oder Enantiomerengemisches versetzt und nach erfolgter Umlagerung nach Abkühlen unter Erhalt einer übersättigten Lösung eine weitere Menge an einheitlichem Diastereomerensalz isoliert. Bei einer bevorzugten mehrfachen Wiederholung von sukzessiver Sättigung, Umlagerung und Abtrennung des Salzes eines Enantiomeren wird der gesamte eingesetzte Dicarbonsäuremonoester in ein Enantiomeres umgewandelt.

Weiter ist bekannt, daß einheitliche Enantiomere unter bestimmten Bedingungen direkt aus dem entsprechenden Racemat zu kristallisieren vermögen (s. Collet et al., Chemical Reviews 80 (1980), 215). Dicarbonsäuremonoester, welche der Spontantrennung zugänglich sind, lassen sich besonders vorteilhaft nach dem erfindungsgemäßen Verfahren ineinander umwandeln. Bei geeigneten Unterschieden in der Löslichkeit zwischen Enantiomeren und Racemat unter den für die Umlagerung angewandten Bedingungen, gelingt die nahezu vollständige Überführung des eingesetzten Dicarbonsäuremonoesters in ein einheitliches Enantiomeres in einem Verfahrensschritt.

Das erfindungsgemäße Verfahren zur Herstellung von Enantiomeren eignet sich insbesondere zur Herstellung von Enantiomeren von cis-1,3-Dibenzyl-2-oxo-imidazolidin-4,5-dicarbonsäuremonoestern. Derartige Monoester sind wertvolle Zwischenprodukte zur Herstellung optisch aktiver Lactone und Thiolactone, wie z. B. beschrieben bei Gerecke et al., Helv. Chim. Acta 53 (1970), 991-999, und weiter zur Herstellung optisch aktiven Biotins, wie z. B. beschrieben in DBP 2 058 234 und DBP 2 331 244.

In der vorliegenden Erfindung steht somit ein sehr vorteilhaftes Verfahren zur einfachen und in hohen Ausbeuten verlaufenden Umlagerung von enantiomeren Dicarbonsäuremonoestern zur Verfügung, das deren Herstellung in hoher optischer Reinheit erlaubt.

## Beispiel 1

5,74 g (15 mMol)(4R, 5S)-cis-1,3-Dibenzyl-5-ethoxycarbonyl-2-oxo-imidazolidin-4-carbonsäure werden unter Zusatz von 0,29 g (3 mMol) Orthophosphorsäure in 60 ml Toluol 32 Stunden unter Rückfluß erhitzt. Nach Abkühlen auf Raumtemperatur wird mit Wasser säurefrei gewaschen, getrocknet, eingeengt und aus Ether/Petrolether kristallisiert; Ausbeute: 4,95 g (86,2 % d. Th.) (4RS, 5RS)-cis-1,3-Dibenzyl-5-ethoxycarbonyl-2-oxo-imidazolin-4-carbonsäure; $[\alpha]_{365}^{20} = 0{,}0°$ (c = 1, DMF).

## Beispiel 2

5,74 g (15 mMol) (4R, 5S)-cis-1,3-Dibenzyl-5-ethoxycarbonyl-2-oxo-imidazolin-4-carbonsäure werden in 60 ml Toluol nach Zusatz von 10 Mol-% Methansulfonsäure 18 Std. unter Rückfluß erhitzt. Nachdem mit Wasser säurefrei gewaschen worden ist, versetzt man in der Wärme mit 4,3 g (15 mMol) (+)-Dehydroabietylamin. Beim Abkühlen auf Raumtemperatur kristallisieren 3,5 g (70 % d. Th. bez. auf vorhandenes Enantiomeres) (4R, 5S)-cis-1,3-Dibenzyl-5-ethoxycarbonyl-2-oxo-imidazolidin-4-carbonsäure-(+)-dehydroabietylaminsalz aus; $[\alpha]_{365}^{20} = +48{,}2°$ (c = 2, Ethanol).

Die nach Abtrennung des voranstehenden Dehydroabietylaminsalzes verbleibende Mutterlauge liefert nach Einengen und Kristallisation 4 g (80 % d. Th. bzw. auf vorhandenes Enantiomeres) (4S, 5R)-cis-1,3-Dibenzyl-5-ethoxycarbonyl-2-oxo-imidazolin-4-carbonsäure-(+)-dehydroabietylaminsalz.

Aus den Dehydroabietylaminsalzen lassen sich die freien Monoester beispielsweise nach dem in der DE-OS 34 31 294 angegebenen Verfahren erhalten.

## Beispiel 3

5,74 g (15 mMol) (4R, 5S)-cis-1,3-Dibenzyl-5-ethoxycarbonyl-2-oxo-imidazolin-4-carbonsaure werden wie in Beispiel 1 angegeben racemisiert. Nachdem mit Wasser säurefrei gewaschen worden ist, versetzt man mit 2,5 g (15 mMol) (+)-Ephedrin, erwärmt unter Rühren kurzzeitig auf 60 °C und beläßt anschließend 24 Stunden bei Raumtemperatur. Das Kristallisat wird unter Nachwaschen mit wenig Toluol

abgesaugt. Durch anschließende Salzspaltung nach bekannten Verfahren (z. B. DE-PS 20 58 248) erhält man 2,4 g (83,6 % d. Th.) (4S, 5R)-cis-1,3-Dibenzyl-5-ethoxycarbonyl-2-oxo-imidazolidin-4-carbonsäure ; $[\alpha]_{365}^{20}$ = — 21,8° (c = 1, DMF).

Aus der nach Abtrennung des (4S, 5R)-Monoesterephedrinsalzes verbleibenden Mutterlauge erhält man 2,7 g (94 % d. Th.) (4R, 5S)-cis-1,3-Dibenzyl-5-ethoxycarbonyl-2-oxo-imidazolin-4-carbonsäure ; $[\alpha]_{365}^{20}$ = + 22,1° (c = 1 ; DMF).

Beispiel 4

8,2 g (20 mMol) (4R, 5S)-cis-1,3-Dibenzyl-5-methoxyethoxycarbonyl-2-oxo-imidazolin-4-carbonsäure (erhältlich aus cis-1,3-Dibenzylhexahydro-1H-furo [3,4-d] imidazol-2,4,6-trion und Ethylenglykolmonomethylether sowie Racematspaltung mittels (+)-Ephedrin ; $[\alpha]_{365}^{20}$ — 6,7° (c = 1, DMF)) werden in Benzol unter Zusatz von Methansulfonsäure nach der in Beispiel 2 angegebenen Arbeitsweise umgelagert. Es werden 96 % d. Th. (4RS, 5RS)-cis-1,3-Dibenzyl-5-methoxyethoxycarbonyl-2-oxo-imidazolidin-4-carbonsäure erhalten ; $[\alpha]_{365}$ = ± 0°.

Beispiel 5

9,0 g (20 mMol) (4R, 5S)-cis-1,3-Dibenzyl-5-benzyloxycarbonyl-2-oxo-imidazolin-4-carbonsäure (DE-OS 35 22 145) werden wie in Beispiel 1 angegeben mit Orthophosphorsäure in Toluol innert 26 Std. umgelagert ; Ausbeute : 94,5 d. Th.

Beispiel 6

9,0 g (20 mMol) (4S, 5R)-cis-1,3-Dibenzyl-5-benzyloxycarbonyl-2-oxo-imidazolidin-4-carbonsäure (DE-OS 35 22 145) werden wie in Beispiel 1 angegeben mit Orthophosphorsäure in Toluol innert 24 Stdn. umgelagert ; Ausbeute : 96 % d. Th.

Beispiel 7

8,6 g (50 mMol) (1R, 2S)-cis-3,3-Dimethyl-2-methoxycarbonylcyclopropancarbonsäure ($[\alpha]_D^{20}$ = 17,5° ; erhalten aus dem Racemat mittels (+)-Ephedrin) werden gemäß Beispiel 1 in Toluol in Gegenwart von Orthophosphorsäure innert 18 Stdn. umgelagert. Ausbeute : 72 % d. Th. ; $[\alpha]_D^{20}$ = 0,0° (c = 1, Chloroform).

Beispiel 8

17,4 g (0,1 Mol) (—)-Ethyl-methylmalonsäuremonoethylester (Kenyon, Ross, J. Chem. Soc. 1951, 3407 ; $[\alpha]_D^{25}$ — 3,5° (c = 5, Chloroform)) werden nach der in Beispiel 2 angegebenen Arbeitsweise unter Zusatz von Methansulfonsäure in Benzol umgelagert. Das erhaltene Produkt zeigt $[\alpha]^{D25}$ = 0,0° (c = 6, Chloroform).

Beispiel 9

2 g (5,1 mMol) (4R, 5S)-cis-1,3-Dibenzyl-5-allyloxycarbonyl-2-oxo-imidazolidin-4-carbonsäure werden unter Stickstoff in 10 ml Tetrahydrofuran gelöst und mit 58 mg (0,05 mMol) Tetrakis-(triphenylphosphin)-palladium (O) sowie 13 mg (0,05 mMol) Triphenylphosphin versetzt und 14 Stunden bei Raumtemperatur gerührt. Anschließend wird filtriert, unter vermindertem Druck vom Lösungsmittel befreit und der Rückstand aus Toluol kristallisiert. Es werden 1,6 g (80 % d. Th.) racemischer cis-1,3-Dibenzyl-5-allyloxycarbonyl-2-oxo-imidazolidin-4-carbonsäure erhalten ; $[\alpha]_{365}^{20}$ = 0,0° (c = 1, Dimethylformamid).

Beispiel 10

2 g (5,1 mMol) (4S, 5R)-cis-1,3-Dibenzyl-5-allyloxycarbonyl-2-oxo-imidazolidin-4-carbonsäure werden unter Stickstoff in 10 ml Tetrahydrofuran gelöst und in eine Lösung von 0,05 mMol Tetrakis-(triphenylphosphin)nickel (O) (erhalten aus Nickel (II)-acetylacetonat, Triphenylphosphin und Diisobutylaluminiumhydrid) in 5 ml Tetrahydrofuran eingetropft. Man läßt 14 Stunden bei Raumtemperatur rühren, filtriert anschließend, befreit unter vermindertem Druck vom Lösungsmittel und kristallisiert den Rückstand aus Toluol um. Es werden 1,7 g (85 % d. Th.) cis-1,3-Dibenzyl-5-allyloxycarbonyl-2-oxo-imidazolidin-4-carbonsäure erhalten ; $[\alpha]_{365}^{20}$ + 0,4° (c = 1, Dimethylformamid).

Beispiel 11

12,0 g (4R, 5S)-cis-1,3-Dibenzyl-5-allyloxycarbonyl-2-oxo-imidazolidin-4-carbonsäure-(+)-ephedri-

niumsalz werden in 500 ml Toluol unter Zusatz von 400 mg Tetrakis-(triphenylphosphin)-palladium (O) und 100 mg Triphenylphosphin 26 Stdn. zum Rückfluß erhitzt. Anschließend rührt man 6 Stdn. bei 0 bis 5°. Das ausgefallene Kristallisat von (4S, 5S)-cis-1,3-Dibenzyl-5-allyloxycarbonyl-2-oxo-imidazolidin-4-carbonsäure-(+)-ephedriniumsalz wird abgetrennt (5,0 g ; 83,3 % d. Th. bez. auf vorhandenes Enantiomeres) und das Filtrat in der Siedehitze mit 5,0 g (4R, 5S)-Ephedriniumsalz versetzt. Nach 25 Stdn. Erhitzen ist die Umlagerung beendet. Bei 5° kristallisieren 4,8 g (80,0 % d. Th.) (4S, 5R)-Ephedriniumsalz. Nach dessen Abtrennung werden erneut 4,8 g (4R, 5S)-Ephedriniumsalz zugesetzt und wie voranstehend angegeben unter Erzielung ähnlich guter Ergebnisse umgelagert.

## Patentansprüche

1. Verfahren zur Umwandlung eines Enantiomeren eines optisch aktiven Dicarbonsäuremonoesters, dessen zugrundeliegende Dicarbonsäure eine Spiegelebene im Molekül aufweist, in das Enantiomere mit umgekehrtem Drehsinn durch Racemisierung und nachfolgende Trennung, dadurch gekennzeichnet, daß man das eingesetzte Enantiomere einer Umesterung unter Protonensäure- oder Nebengruppenmetallsalz-Katalyse unterwirft und das gewünschte Enantiomere aus dem Racemat entweder durch direkte Kristallisation oder nach Überführung mittels einer optisch aktiven Base in ein Diastereomerensalzpaar und dessen Trennung gewinnt.

2. Verfahren nach Anspruch 1, dadurch gekennzeichnet, daß man die Umesterung und die Trennung mehrfach wiederholt.

3. Verfahren nach Anspruch 2, dadurch gekennzeichnet, daß man die Trennung während der Umesterung durchführt.

## Claims

1. Process for converting an enantiomer of an optically active dicarboxylic acid monoester in which the parent dicarboxylic acid has a plane of symmetry in the molecule into the enantiomer having the opposite direction of rotation by racemization and subsequent resolution, characterized in that the enantiomer employed is subjected to a transesterification under catalysis by a proton acid or a subgroup metal salt, and the desired enantiomer is isolated from the racemate either by direct crystallization or after conversion by means of an optically active-base into a pair of diastereomeric salts and the separation of the latter.

2. Process according to claim 1, characterized in that the transesterification and the resolution are carried out several times.

3. Process according to claim 2, characterized in that the resolution is carried out during the transesterification.

## Revendications

1. Procédé pour convertir un énantiomère d'un monoester d'acide dicarboxylique possédant l'activité optique et dont l'acide dicarboxylique présente un plan de symétrie dans la molécule en l'énantiomère à sens de rotation inverse par racémisation suivie d'une séparation, caractérisé en ce que l'on soumet l'énantiomère mis en œuvre à une transestérification avec catalyse par un acide protonique ou un sel de métal des sous-groupes de la Classification Périodique et on isole l'énantiomère recherché à partir du racémate soit par cristallisation directe, soit par conversion à l'aide d'une base possédant l'activité optique en une paire de sels diastéréoisomères qu'on sépare.

2. Procédé selon la revendication 1, caractérisé en ce que l'on répète plusieurs fois la transestérification et la séparation.

3. Procédé selon la revendication 2, caractérisé en ce que la séparation est effectuée au cours de la transestérification.